# EUROPÄISCHE PATENTSCHRIFT

(11) **EP 0 557 876 B1**
(45) Veröffentlichungstag und Bekanntmachung des Hinweises auf die Patenterteilung: **11.12.1996**
(21) Anmeldenummer: 93102450.9
(22) Anmeldetag: 17.02.1993
(51) Int. Cl.: A61K 31/52

(54) **Verwendung von Xanthinderivaten zur Behandlung von Muskelschädigungen nach vollständiger Unterbrechung der Blutzirkulation**
Use of xanthine derivatives for the treatment of muscle damage subsequent to complete interruption of blood circulation
Utilisation de dérivées de la xanthine pour le traitement des dommages musculaires après l'interruption complète de la circulation sanguine

(30) Priorität: 22.02.1992 DE 4205424
(43) Veröffentlichungstag der Anmeldung: 01.09.1993
(73) Patentinhaber: HOECHST AKTIENGESELLSCHAFT, 65926 Frankfurt am Main (DE)
(72) Erfinder: Okyayuz-Baklouti, Ismahan, dr., W-6200 Wiesbaden (DE); Mars, Maurice, Dr., Durban 40001, Natal (ZA); Gregory, Michael Alfred, Link Hills 3652, Natal (ZA)

(56) Entgegenhaltungen:
- EP-A- 0 260 127
- WO-A-87/00523
- GB-A- 1 441 562
- INT.J.MICROCIRC.: CLIN.EXP. Bd. 9, Nr. 4, 1990, Seiten 385 - 400 'Skeletal muscle microcirculation: the effects of limited blood supply and treatment with torbafylline'
- DRUG.DEV.RES. Bd. 20, Nr. 3, 1990, Seiten 291 - 299 'Effects of chronic torbafylline treatment on energy metabolism of ischemic skeletal muscle'
- J.MED. Bd. 21, Nr. 3-4, 1990, Seiten 165 - 80 'Collateral circulation in skeletal muscles: effect of pentoxyfylline and torbafylline'
- COMP.BIOCHEM.PHYSIOL., C: COMP.PHARMACOL.TOXYCOL. Bd. 99C, Nr. 1-2, 1991, Seiten 163 - 168 'The effect of torbafylline on enzyme activities in fast and slow muscles with limited blood supply'
- Eur J.of Pharmacolgy 166,1989,75-86

## Beschreibung

Eine Reihe von Oxoalkyl- und Hydroxyalkylxanthinen wirkt durchblutungsfördernd und kann auch zur Behandlung von Störungen des muskulären Energiestoffwechsels, wie sie insbesondere bei mitochondrialen Myopathien vorliegen eingesetzt werden (EP-A-268 585). Die Wirkung der Xanthinderivate gemäß EP-A-268 585 auf den postischämischen Muskel, wobei die Femoralarterie einer Hinterextremität entweder zeitweise okkludiert wurde oder chronisch verschlossen war, ist bekannt (Int. J. Microcircul. Clin. Exp., 9, 1990, 385 - 400; Drug. Dev. Res., 20, 1990, 291 - 299; J. Med., 21 (3 - 4), 1990, 165 - 180; Comp. Biochem. Physiol., 99 C(1/2), 163 - 168).

Abschnürbinden verwendet man routinemäßig in der Extremitätenchirurgie, um ein blutfreies Operationsfeld zu erhalten. Es gibt zwar Berichte, daß Durchblutungsunterbrechungen bis zu drei Stunden möglich sind, jedoch begrenzen Chirurgen im allgemeinen die Unterbrechung der Durchblutung auf 90 - 120 Minuten (Tountas und Bergman, J. Hand. Surg., 1977, 2 : 31 - 37), da eine längerandauernde Ischämie mit anschließender Wiederdurchblutung zu einer Reihe von Muskelschädigungen, beispielsweise Schwellung der Muskelfasern, Degeneration der Muskelzellen, leichte sensorische und motorische Schäden, Destabilisierungen der Muskelzellmembran, wobei es zu einer Störung des intrazellulären Milieus kommt, oder Schwierigkeiten bei der Rehabilitation führt. Es ist bekannt, daß Schäden nach Wiederdurchblutung von ischämischem Gewebe in Herz, Niere, Darm und Skelettmuskeln auftreten können (McCord, New England J. Med., 1985, 312 (3), 159 - 163). Bisher sind keine Arzneimittel bekannt, die es erlauben, die Zeit der Blutzirkulationsunterbrechung zu verlängern oder die durch die Unterbrechung der Blutzirkulation bedingten Ausfallerscheinungen signifikant zu vermindern.

Es wurde nun gefunden, daß bestimmte Xanthinderivate geeignet sind, die Durchblutungsunterbrechungszeit zu verlängern, die Syndrome zu bessern, die nach Durchblutungsunterbrechung und anschließender Wiederdurchblutung auftreten, sowie die Veränderungen des intrakompartimentalen Drucks zu reduzieren.

Zur Unterbrechung der Blutzirkulation in Geweben, Organen oder Extremitäten kommt es beispielsweise bei Operationen nach Verletzungen der großen Arterien oder Venen, nach Notfalloperationen bei Durchtrennung von Gliedmaßen, Entfernung von Thromben oder anderen Verschlüssen in Arterien oder Venen oder bei Transplantationen z. B. von Niere oder Herz. Je nach Dauer der Operation kann es dann zu mehr oder weniger starken Ausfallerscheinungen am Muskelgewebe kommen, die von leichten Störungen bis zu motorischen Ausfällen, z. B. Lähmungen, reichen können. Teilweise oder kurzzeitige Unterbrechungen der Blutzirkulation führen zu Beginn der Unterbrechung in der Regel nur zu reversiblen Schäden. Es verbleiben intakte Muskelgewebe mit niedrigem extrazellulärem Kaliumgehalt und der Möglichkeit einer vollständigen Regeneration. Überraschenderweise zeigen Versuchstiere mit Gefäßverschluß der hinteren Extremitäten unter Behandlung mit den Xanthinen der Formel I eine schnelle Erholung der Muskelfasern.

Die Erfindung betrifft daher die Verwendung von mindestens einem Xanthinderivat der Formel I 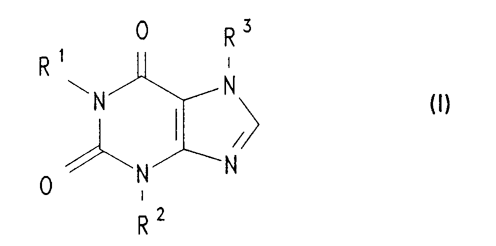 worin mindestens einer der Reste R¹ und R³ eine tertiäre Hydroxyalkylgruppe der Formel Ia 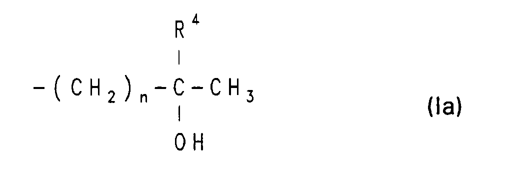 darstellt, in der R⁴ eine Alkylgruppe mit 1, 2 oder 3 C-Atomen und n eine ganze Zahl von 2 - 5 bedeuten, und - falls nur einer der Reste R¹ oder R³ eine solche tertiäre Hydroxyalkylgruppe der Formel Ia darstellt - der andere Rest für ein Wasserstoffatom oder einen aliphatischen Kohlenwasserstoff-Rest R⁵ mit 1 bis 6 C-Atomen steht, dessen Kohlenstoffkette von 1 oder 2 Sauerstoffatomen unterbrochen oder mit einer Oxogruppe oder mit 1 oder 2 Hydroxygruppen substituiert sein kann, wobei diese Oxo- und Hydroxygruppen durch mindestens 2 C-Atome vom Ringstickstoff getrennt sind, und R² eine Alkylgruppe mit 1 - 4 C-Atomen darstellt, zur Herstellung von Arzneimitteln zur Prophylaxe und Reduktion der Muskelschwellung nach Wiederdurchblutung, die nach vollständiger Unterbrechung der Blutzirkulation auftritt.

Bevorzugt werden Xanthinderivate der Formel I verwendet, in denen R² für Methyl oder Ethyl steht und/oder nur einer der beiden Reste R¹ oder R³ die tertiäre Hydroxyalkylgruppe der Formel Ia darstellt. Ferner werden bevorzugt Xanthinderivate der Formel I verwendet, in denen R¹ oder R³ [(ω-1)-Hydroxy-(ω-1)-methyl]-pentyl, -hexyl oder -heptyl bedeuten. Weiterhin werden bevorzugt Xanthinderivate der Formel I verwendet, in denen R¹ [(ω-1)-Hydroxy-(ω-1)-methyl]-pentyl, -hexyl oder -heptyl, R² Methyl oder Ethyl und R³ Wasserstoff, Alkyl oder Alkoxyalkyl mit jeweils 1 - 4 C-Atomen oder Hydroxyalkyl mit 2 - 5 C-Atomen bedeuten. Besonders bevorzugt wird 7-Ethoxymethyl-1-(5-hydroxy-5-methylhexyl)-3-methylxanthin verwendet.

Mit Muskelzellen, Muskelfasern oder Muskelgewebe sind alle Zellen, Gewebe oder Organe gemeint, die zu einer Kontraktion in der Lage sind, insbesondere glatte oder quergestreifte Muskelgewebe, sowie Muskelfasern des Herzens.

Die Xanthinderivate der Formel I werden beispielsweise nach dem folgenden Verfahren hergestellt:
3-Alkylxanthin der Formel II 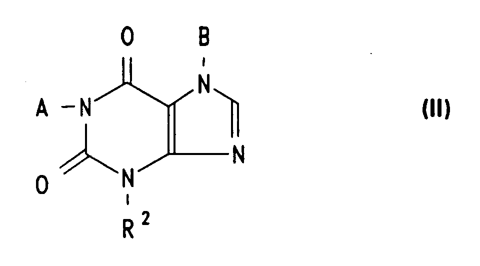 in der R² eine Alkylgruppe mit 1 bis 4 C-Atomen darstellt,
- A: für Wasserstoffatom, R⁵ oder den Rest der Formel Ia und
- B: für Wasserstoffatom, R⁵, Benzyl-/ oder Diphenylmethylrest stehen, wobei aber mindestens einer dieser Reste A und B Wasserstoffatom bedeutet,
werden in Gegenwart mindestens eines basischen Kondensationsmittel oder in Form ihrer Salze in 1- oder/und 7-Position einstufig oder stufenweise mit entsprechenden Alkylierungsmitteln der allgemeinen Formel III

X - Q (III)

in der
- X: Halogenatom oder eine Sulfonsäurester- oder Phosphorsäureestergruppierung und
- Q: eine tertiäre Hydroxyalkylgruppe der Formel Ia, R⁵, Benzyl-/ oder Diphenylmethylrest bedeuten,
unter nachträglicher reduktiver Abspaltung des Restes B, wenn dieser eine Benzyl- oder Diphenylmethylgruppe darstellt, oder gegebenenfalls hydrolytischer Eliminierung eines Alkoxymethyl- oder Alkoxyalkoxymethylrestes aus der Position des Restes B und Reduktion der Ketogruppe zur Alkoholfunktion, wenn A oder B einen Oxoalkylrest bedeutet, bei einer Reaktionstemperatur von 0° C und dem Siedepunkt des jeweils verwendeten Reaktionsmediums alkyliert.

Die obengenannten Umsetzungen erfolgen unter Standardbedingungen auf bekannte Weise (EP-A-268 585, US-A-4 833 146).

Die Ausgangsstoffe der Umsetzungen sind bekannt oder lassen sich nach literaturbekannten Methoden leicht herstellen (EP-A-268 585).

Die Erfindung betrifft auch die Verwendung von Arzneimitteln, die mindestens eine wirksame Menge eines Xanthinderivats der Formel I enthalten, neben pharmazeutisch geeigneten und physiologisch verträglichen Trägerstoffen, Verdünnungsmitteln und/oder anderen Wirk- und Hilfsstoffen.

Die erfindungsgemäßen Arzneimittel werden parenteral, oral, rektal oder gegebenenfalls auch topisch appliziert. Die Applikation erfolgt vor, während oder nach einer Unterbrechung der Blutzirkulation.

Die Erfindung betrifft auch Verfahren zur Herstellung eines Arzneimittels, die dadurch gekennzeichnet sind, daß man mindestens ein Xanthinderivat der Formel I mit einem physiologisch annehmbaren Träger und weiteren geeigneten Wirk-, Zusatz- oder Hilfsstoffen in eine geeignete Darreichungsform bringt.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit-oder Schmiermittel, Geschmackstoffe, Süßungsmittel oder Lösungsvermittler, Verwendung finden. Als häufig verwendete Hilfsstoffe seien z. B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyethylenglykole und Lösungsmittel, wie etwa steriles Wasser und ein- oder mehrwertige Alkohole, z. B. Glycerin, genannt.

Aufgrund der pharmakologischen Eigenschaften der Xanthinderivate können diese Verbindungen bei allen Operationen in der Klinik oder ambulanten Eingriffen angewendet werden, die mit einer Abschnürung und damit Unterbrechung der Blutzirkulation im Muskelgewebe verbunden sind, beispielsweise bei Gefäßverletzungen, bei denen es zu einer längeren Blutleere in den Ektremitäten kommt, wie Abklemmen der Aorta, Embulusentfernung auf chirurgischen Wege oder verspätete Behandlung von abgetrennten großen Blutgefäßen. Die Xanthinderivate können bei der Verminderung von Schäden, die nach der Wiederdurchblutung von Muskelfasern des Herzens auftreten können, eingesetzt werden.

Vorzugsweise werden die pharmazeutischen Präparate in Dosierungseinheiten hergestellt und verabreicht, wobei jede Einheit als aktiven Bestandteil eine bestimmte Dosis mindestens eines Xanthinderivats der Formel I enthält. Bei festen Dosierungseinheiten wie Tabletten, Kapseln, Dragees oder Suppositorien beträgt kann dieses Dosis bis zu etwa 1000 mg, bevorzugt jedoch etwa 100 - 600 mg, und bei Injektionslösungen in Ampullenform bis zu 300 mg, vorzugsweise 20 - 200 mg.

Für die Behandlung eines Patienten (70 kg), bei dem die Blutzirkulation unterbrochen wurde, ist in frühen Phasen eine intravenöse Infussionsbehandlung von 100 - 2000 mg pro Tag und in der späteren Rehabilitationsphase eine orale Verabreichung von 3 mal 400 mg pro Tag insbesondere von 7-Ethoxymethyl-1-(5-Hydroxy-5-methylhexyl)-3-methylxanthin indiziert.

Unter Umständen sind jedoch auch höhere oder niedrigere Dosen angebracht. Die Verabreichung der Dosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Schließlich können die Xanthinderivate der Formel I und/oder deren entsprechende Salze bei der Herstellung der vorgenannten galenischen Zubereitungsformen auch zusammen mit anderen geeigneten Wirkstoffen, beispielsweise Wirkstoffen die freie Sauerstoffradikale abfangen, z. B. 1,5-Hydro-4H-pyrazolo(3,4-d)pyrimidin-4-on, Superoxiddismutase, Dimethylsulfoxid oder Mannitol, Heparin, Ascorbinsäure oder Deferoxamin, formuliert werden.

### Beispiel 1:

### Herstellung von 7-Ethoxymethyl-1-(5-hydroxy-5-methylhexyl)-3-methylxanthin (Verbindung 1)

### a) 7-Ethoxymethyl-3-methylxanthin

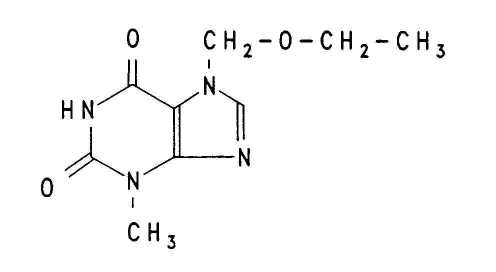

83 g (0,5 mol) 3-Methylxanthin werden in einer Lösung von 20 g (0,5 mol) Natriumhydroxyd in 400 ml Wasser heiß gelöst. Nach Filtration wird das Filtat unter verminderten Druck eingeent, mehrmals Methanol über destilliert und dann trocknet man das Natriumsalz im Hochvakuum. Das getrocknete Salz wird in 1,3 l Dimethylformamid suspendiert, unter Rühren wird die Suspension mit 47,3 g (0,5 mol) Ethoxymethylchlorid versetzt und 18 Stunden bei 110° C gerührt. Anschließend wird heiß filtriert, das Filtrat im Vakuum eingedampft, der Rückstand in 500 ml 2 N Natronlauge gelöst und zur Entfernung von als Nebenprodukt gebildeten 1,7-dialkylierten 3-Methylxanthin mit Chloroform ausgeschüttelt. Die alkalische wäßrige Lösung bringt man mit 2 N Salzsäure unter Rühren auf pH 9, trennt das gebildete Kristallisat ab, wäscht zunächst mit Wasser das Kristallisat chlorid-frei und dann mit Methanol und trocknet im Vakuum.
- Ausbeute:: 77,6 g (69,2 % der Theorie)
Schmelzpunkt: 263 - 264° C
C₉H₁₂N₄O₃ (MG = 224,2)

### b) 7-Ethoxymethyl-1-(5-hydroxy-5-methylhexyl)-3-methylxanthin

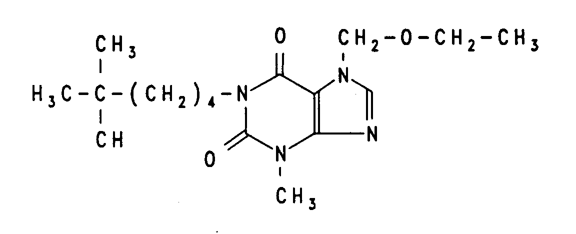

11,2 g (0,05 mol) 7-Ethoxymethyl-3-methylxanthin werden in 300 ml Dimethylformamid mit 7,5 g (0,054 mol) Kaliumcarbonat und 8,2 g (0,054 mol) 1-Chlor-5-hydroxy-5-methylhexan versetzt und unter Rühren 5 Stunden auf 110° C erhitzt. Man saugt heiß ab, engt im Vakuum ein, nimmt den Rückstand in Chloroform auf, wäscht zunächst mit 1 N Natronlauge und dann mit Wasser neutral, trocknet über Natriumsulfat, destilliert das Lösungsmittel unter vermindertem Druck ab und kristallisiert den Rückstand aus Diisopropylether unter Zusatz von Essigsäureethylester und Petrolether um.
- Ausbeute:: 14,1 g (83,3 % der Theorie)
Schmelzpunkt: 102 - 103° C
C₁₆H₂₆N₄O₄ (MG = 338,4)

| | | | | |
|---|---|---|---|---|
| Analyse: | Berechnet: | C 56,79 % | H 7,74 % | N 16,56 % |
| | Gefunden: | C 56,76 % | H 7,82 % | N 16,59 % |

### Beispiel 2:

### Herstellung eines Arzneimittels

Ein injizierbares Arzneimittel wird wie folgt hergestellt:

10 g der Verbindung aus Beispiel 1 wird in Wasser durch Rühren und langsames Erwärmen gelöst und mit Wasser auf 1000 ml Endvolumen aufgefüllt. Die erhaltene Lösung wird durch eine 0,2 µm Membranfilter filtriert und in Ampullen mit 5 oder 10 ml Inhalt gefüllt, die nach dem Zuschmelzen für 15 Minuten bei 120° C sterilisiert werden.

Tabletten, die 250 mg der Verbindung aus Beispiel 1 enthalten, werden auf übliche Weise durch Mischen von 200 g der Verbindung aus Beispiel 1, 150 g Lactose, 30 g Stärke, 10 g Crospovidon, 10 g Talk, 2 g kolloidales Siliziumdioxid und 1,5 g Magnesiumstearinat hergestellt. Die Tabletten erhalten einen Mantel aus einer wässrigen Suspension bestehend aus 40 g Hydroxypropylmethylcellulose, 2 g Polyethylenglycol mit einem durchschnittlichen Molekulargewicht von 6000, 3,5 g Titandioxid, 3 g Talk und 451,5 g Wasser. Pro Tablette werden etwa 5 bis 10 mg Feststoffe als Überzug aufgebracht.

### Beispiel 3:

Zwei Gruppen von acht erwachsenen, männlichen südafrikanischen Meerkatzen (Cercopithecus pygerythrus) werden untersucht. Die Anasthäsie der Tiere erfolgt intramuskulär mit 15 mg/kg Ketamin (Warner Lambert) und wird intravenös mit Thiopenton Natrium (25 mg/kg, May und Baker) aufrecht erhalten. Eine hintere Extremität wird durch eine pneumatische Abschnürbinde und eine Esmarch Bandage blutleer gemacht. Die Unterbrechung der Blutzirklation wird drei Stunden aufrechterhalten, dann wird die Unterbrechung beendet und die Tiere können aus der Betäubung aufwachen. Die Entnahme der Muskelgewebeproben erfolgt unter Betäubung (15 mg/kg Ketamin).

20 mg/kg Verbindung 1 wird intravenös über 30 Minuten den Tieren appliziert bevor die Unterbrechung der Blutzirkulation erfolgt.

### Muskelpräparation:

Das entnommene Muskelgewebe wird sofort in Isopentan bei -183° C eingefroren. Die Präparation des Muskelgewebes erfolgt wie bei Dubowitz (Dubowitz et al. Muscle Biopsy A practical Approach, London, Bailliere Tindall, 1985, S. 82 - 128) beschrieben. Mikroskopische Schnitte werden mit Muskelfaser-ATPase für 30 Minuten bei pH 9,4 gefärbt, eingebettet und im Lichtmikroskop untersucht. Die Durchmesser von wenigstens 200 Muskelfasern werden mit einem computerisierten Video-Image-Analysator ausgewertet.

Der durchschnittliche Durchmesser jedes Muskelfasertyps wird in Tabelle 1 beschrieben.

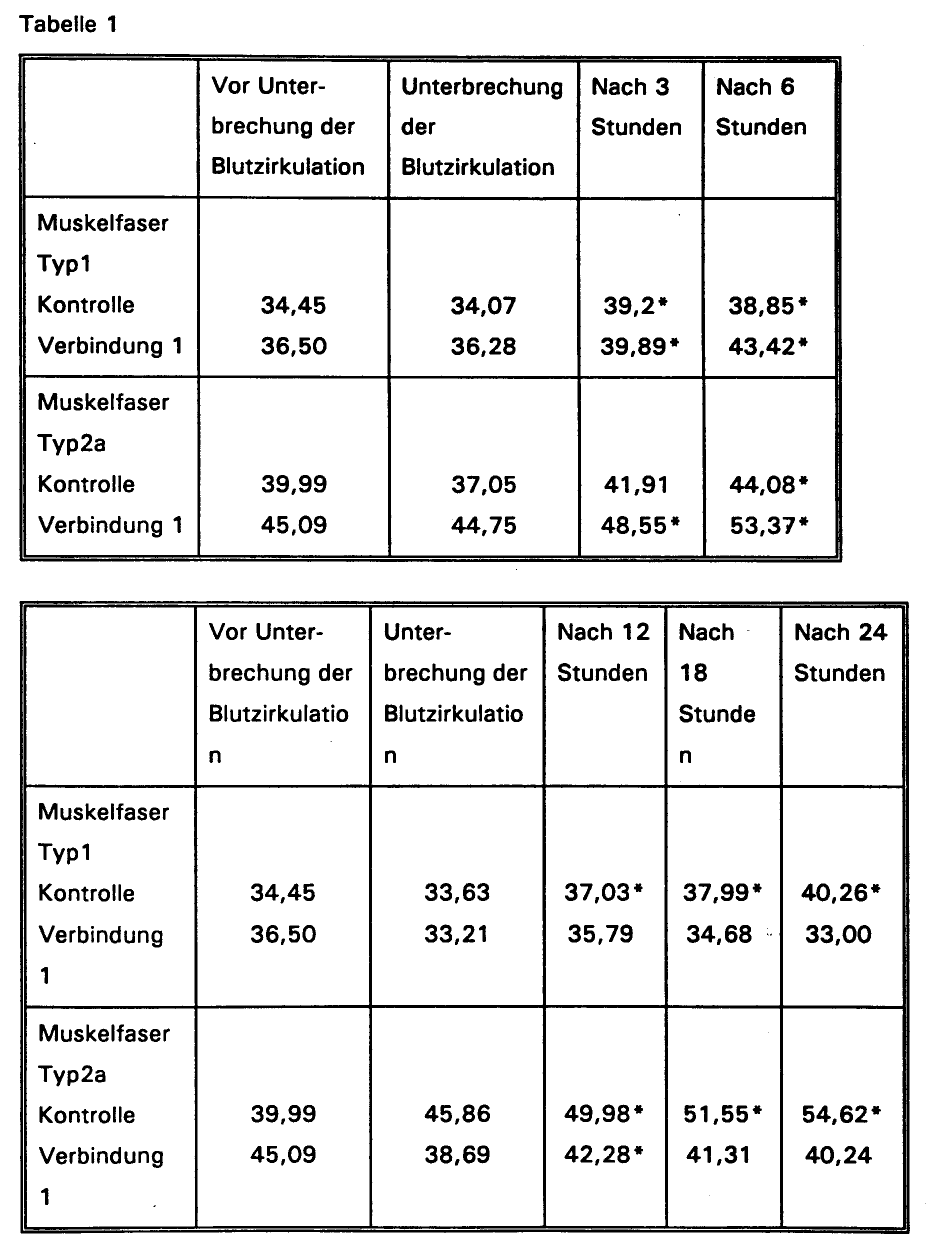

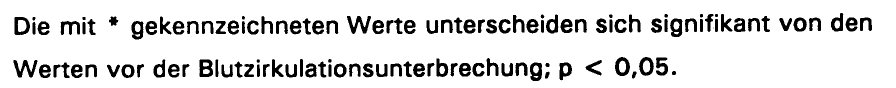

Die Werte in Tabelle 1 zeigen, daß die Muskelgewebe der mit Verbindung 1 behandelten Tiere eine signifikant verringerte Schwellung der Muskelfasern vom Typ 1 und 2a nach 18 - 24 Stunden Wiederdurchblutung aufweisen.

## Patentansprüche

1. Verwendung von mindestens einem Xanthinderivat der Formel I 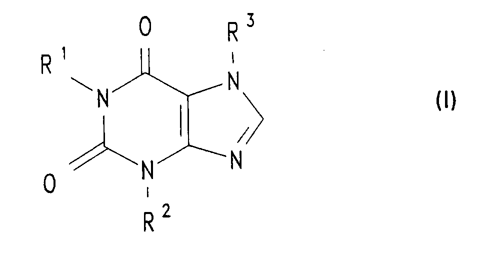 worin mindestens einer der Reste R¹ und R³ eine tertiäre Hydroxyalkylgruppe der Formel Ia 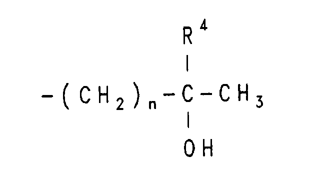 darstellt, in der R⁴ eine Alkylgruppe mit 1, 2 oder 3 C-Atomen und n eine ganze Zahl von 2 - 5 bedeuten, und - falls nur einer der Reste R¹ oder R³ eine solche tertiäre Hydroxyalkylgruppe der Formel Ia darstellt - der andere Rest für ein Wasserstoffatom oder einen aliphatischen Kohlenwasserstoff-Rest R⁵ mit 1 bis 6 C-Atomen steht, dessen Kohlenstoffkette von 1 oder 2 Sauerstoffatomen unterbrochen oder mit einer Oxogruppe oder 1 oder 2 Hydroxygruppen substituiert sein kann, wobei diese Oxo- und Hydroxygruppen durch mindestens 2 C-Atome vom Ringstickstoff getrennt sind, und R² eine Alkylgruppe mit 1 - 4 C-Atomen darstellt, zur Herstellung von Arzneimitteln zur Prophylaxe und Reduktion der Muskelschwellung nach Wiederdurchblutung, die nach vollständiger Unterbrechung der Blutzirkulation auftritt.

2. Verwendung gemäß Anspruch 1, dadurch gekennzeichnet, daß man 7-Ethoxymethyl-1-(5-hydroxy-5-methylhexyl)-3-methylxanthin einsetzt.

3. Verwendung gemäß der Ansprüche 1 und 2 zur Herstellung von Arzneimitteln zur Verlängerung der Operationszeit nach vollständiger Unterbrechung der Blutzirkulation.

4. Verwendung gemäß einem oder mehreren der Ansprüche 1 - 3 vor, nach und während der Operation, bei der es zur vollständigen Unterbrechung der Blutzirkulation kommt.

5. Verwendung gemäß Anspruch 1, dadurch gekennzeichnet, daß mindestens ein Xanthinderivat der Formel I, bei dem R² für Methyl oder Ethyl steht und/oder nur einer der beiden Reste R¹ oder R³ die im Anspruch 1 definierte tertiäre Hydroxyalkylgruppe der Formel Ia darstellt, eingesetzt wird.

6. Verwendung gemäß Anspruch 1, dadurch gekennzeichnet, daß mindestens ein Xanthinderivat der Formel I, bei dem R¹ oder R³ [(ω-1)-Hydroxy-(ω-1)-methyl]-pentyl, -hexyl oder -heptyl bedeuten, eingesetzt wird.

7. Verwendung gemäß Anspruch 1, dadurch gekennzeichnet, daß mindestens ein Xanthinderivat der Formel I, bei dem R¹ [(ω-1)-Hydroxy-(ω-1)-methyl]-pentyl, -hexyl oder -heptyl, R² Methyl oder Ethyl und R³ Wasserstoff, Alkyl oder Alkoxyalkyl mit jeweils 1 - 4 C-Atomen oder Hydroxyalkyl mit 2 - 5 C-Atomen bedeuten, eingesetzt wird.

## Claims

1. The use of at least one xanthine derivative of the formula I 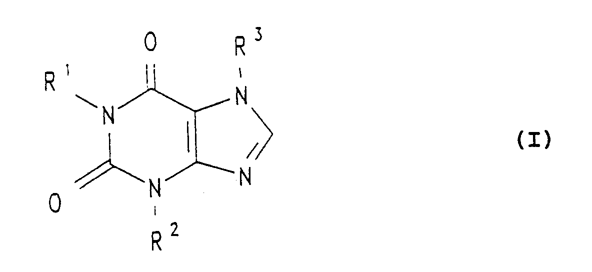 in which at least one of the radicals R¹ and R³ is a tertiary hydroxyalkyl group of the formula Ia 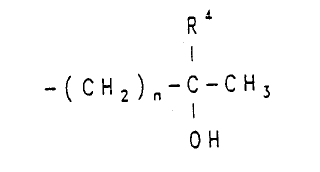 in which R⁴ is an alkyl group with 1, 2 or 3 carbon atoms and n is an integer of 2 - 5 and - if only one of the radicals R¹ or R³ is such a tertiary hydroxyalkyl group of the formula Ia - the other radical is a hydrogen atom or an aliphatic hydrocarbon radical R⁵ which has 1 to 6 carbon atoms and whose carbon chain can be interrupted by 1 or 2 oxygen atoms or substituted by an oxo group or by 1 or 2 hydroxyl groups, these oxo and hydroxyl groups being separated by at least 2 carbon atoms from the ring nitrogen, and R² is an alkyl group with 1 - 4 carbon atoms, for the preparation of pharmaceuticals for the prophylaxis and reduction of muscular swelling after restoration of blood flow which occurs after complete interruption of the blood circulation.

2. The use as claimed in claim 1, wherein 7-ethoxymethyl-1-(5-hydroxy-5-methylhexyl)-3-methylxanthine is employed.

3. The use as claimed in claims 1 and 2 for the preparation of pharmaceuticals for prolonging the operation time after complete interruption of the blood circulation.

4. The use as claimed in one or more of claims 1 - 3 before, after and during the operation in which there is complete interruption of the blood circulation.

5. The use as claimed in claim 1, wherein at least one xanthine derivative of the formula I in which R² is methyl or ethyl and/or only one of the two radicals R¹ or R³ is the tertiary hydroxyalkyl group of the formula Ia defined in claim 1 is employed.

6. The use as claimed in claim 1, wherein at least one xanthine derivative of the formula I in which R¹ or R³ is [(ω-1)-hydroxy-(ω-1)-methyl]-pentyl, -hexyl or -heptyl is employed.

7. The use as claimed in claim 1, wherein at least one xanthine derivative of the formula I in which R¹ is [(ω-1)-hydroxy-(ω-1)-methyl]-pentyl, -hexyl or -heptyl, R² is methyl or ethyl and R³ is hydrogen, alkyl or alkoxyalkyl with, in each case, 1 - 4 carbon atoms or hydroxyalkyl with 2 - 5 carbon atoms is employed.

## Revendications

1. Utilisation d'au moins un dérivé de xanthine de formule I 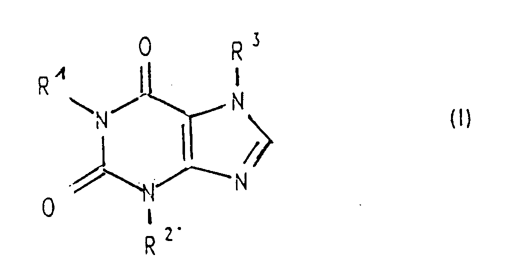 dans laquelle au moins l'un des radicaux R¹ et R³ représente un groupe hydroxyalkyle tertiaire de formule Ia 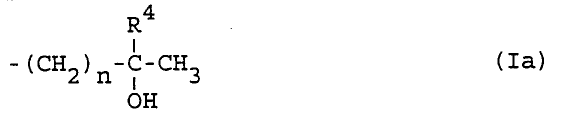 dans laquelle R⁴ représente un groupe alkyle ayant 1, 2 ou 3 atomes de carbone et n représente un nombre entier allant de 2 à 5, et - lorsqu'un seul des radicaux R¹ et R³ représente un tel groupe hydroxyalkyle tertiaire de formule Ia - l'autre radical représente un atome d'hydrogène ou un radical hydrocarboné aliphatique R⁵ ayant de 1 à 6 atomes de carbone, dont la chaîne carbonée peut être interrompue par 1 à 2 atomes d'oxygène ou être substituée par un groupe oxo ou par 1 ou 2 groupes hydroxy, ces groupes oxo et hydroxy étant séparés par au moins 2 atomes de carbone de l'atome d'azote présent dans le cycle, et R² représente un groupe alkyle ayant de 1 à 4 atomes de carbone,
pour la fabrication de médicaments destinés à la prophylaxie et à la réduction du gonflement musculaire après réirrigation, apparaissant après une interruption totale de la circulation sanguine.

2. Utilisation selon la revendication 1, caractérisée en ce que l'on utlise la 7-éthoxyméthyl-1-(5-hydroxy-5-méthylhexyl)-3-méthylxanthine.

3. Utilisation selon les revendications 1 et 2, pour la fabrication de médicaments destinés à prolonger le temps d'une opération après interruption totale de la circulation sanguine.

4. Utilisation selon une ou plusieurs des revendications 1 à 3, avant, après et pendant l'opération dans laquelle on aboutit à une interruption totale de la circulation sanguine.

5. Utilisation selon la revendication 1, caractérisée en ce que l'on utilise au moins un dérivé de xanthine de formule I dans lequel R² représente le groupe méthyle ou éthyle et/ou un seul des radicaux R¹ et R³ représente le groupe hydroxyalkyle tertiaire de formule Ia, défini dans la revendication 1.

6. Utilisation selon la revendication 1, caractérisée en ce que l'on utilise au moins un dérivé de xanthine de formule I dans lequel R¹ ou R³ représente le groupe [(ω-1)-hydroxy-(ω-1)-méthyl]pentyle, -hexyle ou -heptyle.

7. Utilisation selon la revendication 1, caractérisée en ce que l'on utilise au moins un dérivé de xanthine de formule I dans lequel R¹ représente le groupe [(ω-1)-hydroxy-(ω-1)-méthyl]pentyle, -hexyle ou -heptyle, R² représente le groupe méthyle ou éthyle, et R³ représente un atome d'hydrogène ou un groupe alkyle ou alcoxyalkyle ayant chacun de 1 à 4 atomes de carbone, ou un groupe hydroxyalkyle ayant de 2 à 5 atomes de carbone.
